Europäisches Patentamt

(19) European Patent Office

Office européen des brevets

(11) Veröffentlichungsnummer: **0 340 587**
**A2**

(12) **EUROPÄISCHE PATENTANMELDUNG**

(21) Anmeldenummer: 89107368.6

(22) Anmeldetag: 24.04.89

(51) Int. Cl.⁴ **C07C 29/10 , C07C 31/20 , C07C 69/675 , C07C 67/31 , C07C 67/29**

(30) Priorität: 02.05.88 DE 3814850

(43) Veröffentlichungstag der Anmeldung:
08.11.89 Patentblatt 89/45

(84) Benannte Vertragsstaaten:
**AT BE CH DE ES FR GB IT LI NL SE**

(71) Anmelder: **Henkel Kommanditgesellschaft auf Aktien**
**Postfach 1100 Henkelstrasse 67**
**D-4000 Düsseldorf 1(DE)**

(72) Erfinder: **Stoll, Gerhard, Dr.**
**Danziger Strasse 69**
**D-4052 Korschenbroich 1(DE)**
Erfinder: **Grundt, Elke**
**Feldstrasse 10 a**
**D-4010 Hilden(DE)**
Erfinder: **Höfer, Rainer, Dr.**
**Klever Strasse 31**
**D-4000 Düsseldorf 30(DE)**
Erfinder: **Kluth, Hermann**
**Degerstrasse 48**
**D-4000 Düsseldorf(DE)**
Erfinder: **Kihn-Botulinski, Martina, Dr.**
**Kappeler Strasse 3 a**
**D-4000 Düsseldorf 13(DE)**
Erfinder: **Grützmacher, Roland**
**Heinrich-Heine-Strasse 2**
**D-5603 Wülfrath(DE)**

(54) Verfahren zur Herstellung von langkettigen Kohlenstoffverbindungen mit vicinalen Diolgruppen.

(57) Ein Verfahren zur Herstellung von langkettigen Kohlenstoffverbindungen mit vicinalen Diolgruppen durch Umsetzung von Epoxyverbindungen aus der Gruppe der epoxidierten ungesättigten $C_{16}$-$C_{22}$-Fettalkohole, der epoxidierten Ester von ungesättigten $C_{16}$-$C_{22}$-Fettalkoholen mit $C_2$-$C_5$-Monocarbonsäuren, der epoxidierten ungesättigten $C_{16}$-$C_{22}$-Fettsäureester, der epoxidierten ungesättigten $C_{16}$-$C_{22}$-Fettsäureglyceride und der epoxidierten $C_8$-$C_{22}$-Alkene mit Wasser liefert in glatter Reaktion die gewünschten vicinalen Diole, wenn man die Epoxyverbindungen bei Temperaturen von 180 bis 250°C und einem Druck von 15 bis 40 bar in Abwesenheit von die Ringöffnung von Oxirangruppen katalysierenden Verbindungen und von organischen Lösemitteln hydrolysiert.

## Verfahren zur Herstellung von langkettigen Kohlenstoffverbindungen mit vicinalen Diolgruppen.

Die Erfindung betrifft ein Verfahren zur Herstellung von langkettigen Kohlenstoffverbindungen mit vicinalen Diolgruppen durch Umsetzung von Epoxyverbindungen aus der Gruppe der epoxidierten, ungesättigten $C_{16}$-$C_{22}$-Fettalkohole, der epoxidierten Ester von ungesättigten $C_{16}$-$C_{22}$-Fettalkoholen mit $C_2$-$C_5$-Monocarbonsäuren, der epoxidierten ungesättigten $C_{16}$-$C_{22}$-Fettsäureester, der epoxidierten ungesättigten $C_{16}$-$C_{22}$-Fettsäureglyceride und der epoxidierten $C_8$-$C_{22}$-Alkene mit Wasser.

Die Herstellung vicinaler Diole aus Epoxiden durch Hydrolyse mit Wasser erfolgte bisher in Gegenwart saurer oder basischer Katalysatoren, z.B. von Schwefelsäure, Natronlauge oder Aminen, in Substanz oder unter Zusatz mit Wasser mischbarer Lösemittel wie Dioxan, Diglykoldiethern und dergleichen. Diese Verfahren sind nachteilig, weil die erforderliche Abtrennung der Katalysatoren nach der Umsetzung zu einer Salzbelastung im Abwasser führt; gegebenenfalls vorhandene organische Lösemittel müssen in einem zusätzlichen Arbeitsschritt sorgfältig entfernt werden. Ein weiteres Verfahren zur Herstellung von vicinalen Diolen besteht in der Oxidation von Verbindungen mit olefinischen Doppelbindungen mit Permanganat, Osmiumtetroxid oder dergleichen und dürfte für eine großtechnische Anwendung ungeeignet sein.

Es wurde nun gefunden, daß man langkettige Kohlenstoffverbindungen mit vicinalen Diolgruppen in besonders einfacher und wirtschaftlicher sowie abwasserschonender Weise erhalten kann, wenn man die in technischen Maßstäben zur Verfügung stehenden Epoxyverbindungen der eingangs genannten Art bei Temperaturen von 180 bis 250° C und einem Druck von 15 bis 40 bar in Abwesenheit von die Ringöffnung von Oxirangruppen katalysierenden Verbindungen und von organischen Lösemitteln hydrolysiert.

Zwar ist aus Ullmann, Enzyklopädie der technischen Chemie, 3. Auflage, Seite 146 (1953) bekannt, daß Ethylenoxid bei 160 bis 180° C unter Druck mit Wasser Ethylenglykol bildet; vom Ethylenoxid ist jedoch bekannt, daß es wesentlich reaktiver als Epoxide höherer Alkene ist. Zudem bleibt die Umsetzung von Ethylenoxid mit Wasser unter den vorgenannten Bedingungen nicht auf der Stufe des Ethylenglykols stehen; vielmehr reagiert Ethylenglykol mit weiterem Ethylenoxid, so daß letzlich Polyethylenglykole erhalten werden. Es konnte daher nicht erwartet werden, daß die eingangs erwähnten höheren Epoxyverbindungen unter den Bedingungen des Verfahrens der Erfindung in hohen Ausbeuten die erwünschten vicinalen Diolverbindungen ergeben.

Durch das Verfahren der Erfindung werden die Nachteile des Standes der Technik vermieden; d.h. es wird ausschließlich mit Wasser als Hydrolysereagenz gearbeitet, so daß die Entfernung von Neutralisationsprodukten der bisher eingesetzten Katalysatoren sowie die Beseitigung von Hilfslösemitteln nicht erforderlich ist.

Gemäß vorteilhaften Ausführungsformen des Verfahrens der Erfindung beträgt die Reaktionsdauer 2 bis 8 Stunden, die Hydrolysetemperatur 220 bis 235° C und das eingesetzte Gewichtsverhältnis von Epoxyverbindungen zu Wasser 1:0,13 bis 1:10, insbesondere 1:5 bis 1:8.

Als Ausgangsverbindungen in Verfahren der Erfindung können die folgenden eingesetzt werden:

1. Epoxidierte ungesättigte $C_{16}$-$C_{22}$-Fettalkohole.

Hierbei handelt es sich um die großtechnisch hergestellten Epoxidationsprodukte von ungesättigten Fettalkoholen pflanzlichen, tierischen und/oder synthetischen Ursprungs, wie sie z.B. durch Hydrierung der entsprechenden Fettsäureester und anschließende Epoxidation erhalten werden können. In den meisten Fällen stellen die epoxidierten ungesättigten Fettalkohole technische Gemische dar, wie sie aus Ölsäuren, Sojafettsäuren, Erucasäuren, Rübölfettsäuren, Leinölfettsäuren, Safflorfettsäuren, Sonnenblumenfettsäuren, Baumwollsaatfettsäuren und Rindertalgfettsäuren, z.B. durch Hydrierung der entsprechenden Methylester, erhältlich sind.

2. Epoxidierte Ester von ungesättigten $C_{16}$-$C_{22}$-Fettalkoholen mit $C_2$-$C_5$-Monocarbonsäuren.

Diese Verbindungen leiten sich von den vorgenannten Fettalkoholen ab und sind mit Monocarbonsäuren, z.B. Propionsäure, Buttersäure und Valeriansäure, insbesondere jedoch mit Essigsäure, verestert.

3. Epoxidierte ungesättigte $C_{16}$-$C_{22}$-Fettsäureester.

Diese Verbindungen leiten sich von den vorgenannten ungesättigten Fettsäuren ab und sind im allgemeinen mit $C_1$-$C_8$-Monoalkanolen, insbesondere Methanol, verestert.

4. Epoxidierte ungesättigte $C_{16}$-$C_{22}$-Fettsäureglyceride.

Auch diese Verbindungen leiten sich von den oben genannten natürlichen und/oder synthetischen Fettsäuren ab; geeignet sind epoxidierte Mono-, Di- und Triglyceride einschließlich Mischungen derselben, insbesondere Triglyceride der verschiedensten natürlichen Provenienzen.

5. Epoxidierte C$_8$-C$_{28}$-Alkene.

Diese Verbindungen werden insbesondere in Form ihrer endständig ungesättigten Vertreter großtechnisch hergestellt; typische Vertreter sind epoxidierte Octene, Decene, Dodecene, Tetradecene, Hexadecene, Octadecene, Eicosene und dergleichen.

Die Anwendung des Verfahrens der Erfindung ist nicht auf Verbindungen der vorstehend genannten Art beschränkt, die im Molekül nur eine epoxidierte olefinische Doppelbindung aufweisen; es können vielmehr jedoch - häufig auch mit Vorteil - solche Verbindungen eingesetzt werden, die 2 bis 3 oder bis zu 4 olefinische Doppelbindungen bzw. Epoxyfunktionen nach der Epoxidation aufweisen.

Ein weiterer Vorteil des Verfahrens der Erfindung ist, daß epoxidierte Ester von ungesättigten C$_{16}$-C$_{22}$-Fettalkoholen mit C$_2$-C$_5$-Monocarbonsäuren und epoxidierte ungesättigte C$_{16}$-C$_{22}$-Fettsäureester einschließlich der Glyceride derselben nicht nur unter Öffnung der Oxiranringe, sondern bei geeigneter Reaktionsführung gleichzeitig auch unter Verseifung der Esterfunktion reagieren, so daß man in einem Schritt die entsprechenden Polyole bzw. Polyhydroxycarbonsäuren erhält.

Die Erfindung wird im folgenden anhand bevorzugter Ausführungsbeispiele näher erläutert.

Beispiel 1.

D,L-1,2-Dodecandiol.

15 kg 1,2-Epoxydodecan (Epoxy-O-gehalt = 8,00 %) wurden in einem Autoklaven mit 90 kg Wasser 6 h bei 220° C und einem Eigendruck von 22 bar gerührt. Nach Entfernung der Wasserphase wurde die organische Phase bei 5 Torr destilliert. Man erhielt 13 kg eines festen, farblosen Produkts.

Analytische Daten:

OHZ: 525 (theor.: 555)
SZ: 0,6
Epoxy-O-Gehalt: 0

Beispiel 2.

D,L-1,2-Tetradecandiol.

Die Herstellung erfolgte analog zu dem Beispiel 1 aus 1,2-Epoxytetradecan, wobei das Reaktionsprodukt jedoch nicht destilliert, sondern lediglich bei 90° C im Vakuum getrocknet wurde.

Analytische Daten:

OHZ: 397,3
SZ: 0,6
VZ: 9,9
JZ: 0,02
Epoxy-O-Gehalt: 0,02 %.

Beispiel 3.

D,L-1,2-Hexadecandiol.

Die Herstellung erfolgte analog zu dem Beispiel 1 aus 5 kg Epoxyhexadecan. Man erhielt 3,2 kg eines festen, farblosen Diols.

Analytische Daten:

OHZ: 428,3
SZ: 0,2
VZ: 2,3
JZ: 0,1
Epoxy-O-Gehalt: 0

Beispiel 4.

Hydrolyse von epoxidiertem Oleylalkohol.

Es wurde ein epoxidierter Oleylalkohol (0 bis 2 % C$_{14}$, 2 bis 10 % C$_{16}$, 87 bis 95 % C$_{18}$, 0 bis 3 % C$_{20}$) mit einem Epoxid-O-Gehalt von 4,00 % eingesetzt. 14 kg dieses epoxidierten Oleylalkohols wurden in der im Beispiel 1 beschriebenen Weise mit 42 kg Wasser umgesetzt. Nach dem Trocknen im Vakuum ohne Destillation erhielt man 13,4 kg eines beigefarbenen, festen Produkts.

Analytische Daten:

OHZ: 413,9
JZ: 14,7
VZ: 9,8
SZ: 1,2
Epoxy-O-Gehalt: 0
Wasser: 0,08 % (nach Fischer).

Beispiel 5.

Hydrolyse von epoxidiertem Sojaöl.

Ein Teil epoxidierten Sojaöls (Epoxy-O-Gehalt 6,8 %) wurde mit 5,5 Gew.-Teilen Wasser 5,5 h im Autoklaven unter Rühren auf 220°C unter 22 bar Eigendruck erhitzt. Nach dem Entfernen des Wassers und Trocknen der organischen Phase bei 100°C im Wasserstrahlvakuum erhielt man eine gelbbraune, klare, mäßig viskose Flüssigkeit.

Analytische Daten:

Epoxy-O-Gehalt: 0,12 %
SZ: 10,0
VZ: 179,9
OHZ: 274,0
JZ: 9,6
Viskosität: ca. 60 000 mPas
freies Glycerin in der Wasserphase: 0,06 %.

Beispiel 6.

Partielle Druckspaltung eines epoxidierten Sojaöls:

Ein Teil epoxidiertes Sojaöl (Epoxy-O-Gehalt: 6,80 %) wurde mit 3 Gew.-Teilen Wasser im Autoklaven 3 h bei 220°C gerührt. Nach Entfernen der Wasserphase und dem Trocknen der organischen Phase bei 90°C im Wasserstrahlvakuum erhielt man eine gelbe, klare Flüssigkeit.

Analytische Daten:

Epoxy-O-Gehalt: 4,69
SZ: 0,50
VZ: 178,5
JZ: 5,5

**Ansprüche**

1. Verfahren zur Herstellung von langkettigen Kohlenstoffverbindungen mit vicinalen Diolgruppen durch Umsetzung von Epoxyverbindungen aus der Gruppe der epoxidierten ungesättigten $C_{16}$-$C_{22}$-Fettalkohole, der epoxidierten Ester von ungesättigten $C_{16}$-$C_{22}$-Fettalkoholen mit $C_2$-$C_5$-Monocarbonsäuren, der epoxidierten ungesättigten $C_{16}$-$C_{22}$-Fettsäureester, der epoxidierten ungesättigten $C_{16}$-$C_{22}$-Fettsäureglyceride und der epoxidierten $C_8$-$C_{28}$-Alkene mit Wasser, dadurch gekennzeichnet, daß man die Epoxyverbindungen bei Temperaturen von 180 bis 250°C und einem Druck von 15 bis 40 bar in Abwesenheit von die Ringöffnung von Oxirangruppen katalysierenden Verbindungen und von organischen Lösemitteln hydrolysiert.

2. Verfahren nach Anspruch 1, gekennzeichnet durch eine Reaktionsdauer von 2 bis 8 Stunden.

3. Verfahren nach Anspruch 1 oder 2, gekennzeichnet durch eine Hydrolysetemperatur von 210 bis 235°C.

4. Verfahren nach mindestens einem der Ansprüche 1 bis 3, gekennzeichnet durch ein Gewichtsverhältnis von Epoxyverbindungen zu Wasser von 1:0,13 bis 1:10, insbesondere von 1:5 bis 1:8.